# EUROPÄISCHE PATENTANMELDUNG

(11) **EP 1 795 116 A1**
(43) Veröffentlichungstag der Anmeldung: **13.06.2007**
(21) Anmeldenummer: 05027063.6
(22) Anmeldetag: 12.12.2005
(51) Int. Cl.: A61B 5/00, A61M 5/00

(54) **System mit einem tragbaren Patientengerät und externem Bedienteil**

(71) Anmelder: F. HOFFMANN-LA ROCHE AG, 4070 Basel (CH); Roche Diagnostics GmbH, 68305 Mannheim (DE)
(72) Erfinder: Frikart, Marcel, 3012 Bern (CH); Jungen, Markus, 3063 Bolligen (CH); Ehrsam, Matthias, CH-3065 Bolligen (CH); Friedli, Kurt, 3421 Lyssach (CH)
(74) Vertreter: Blum, Rudolf Emil

(57) **Zusammenfassung**

Ein Patientensystem (1) besteht aus einem am Körper eines Patienten tragbaren ersten Patientengerät (2) und einem vom ersten Patientengerät (2) entfernt angeordneten zweiten Patientengerät (4). Die Bedienoberfläche des ersten Patientengerätes (2) ist vom diesem abgesetzt auf dem zweiten Patientengerät (4) darstellbar.

## Beschreibung

### Technisches Gebiet

Die Erfindung betrifft allgemein ein System mit einem tragbaren Patientengerät. Die Erfindung betrifft insbesondere die Bedienung des Patientengerätes.

### Stand der Technik

Tragbare Patientengeräte sind für eine Vielzahl von Anwendungen bekannt, beispielsweise zur Blutdruckmessung, Pulsmessung, EKG Aufnahme und Medikamentzufuhr. Diese Patientengeräte sind mittlerweile wichtige Hilfsmittel bei der Diagnose und Therapie, die es den Patienten ermöglichen, dem gewohnten Alltag ohne grosse Einschränkungen nachzugehen, den Ärzten aber beispielsweise aussagekräftigere Messungen unter Alltagsbedingen erlauben.

Ein beispielhaftes Patientengerät zur Medikamentzufuhr ist eine Insulinpumpe, z. B. eine Accu-Chek® Insulinpumpe der Roche Diagnostics GmbH, Deutschland. Ein Patient trägt die Insulinpumpe in der Regel am Körper. Über einen dünnen Schlauch, dessen Kanüle unter der Haut sitzt, gibt die Insulinpumpe kontinuierlich Insulin an den Körper ab. Mikroprozessoren steuern einen Motor, der z. B. alle drei Minuten über eine Gewindestange einen Stopfen in eine Insulinampulle bewegt. Diese Bewegung, die der jeweils programmierten Basalrate, d. h. des Insulingrundbedarfs des Patienten, entspricht, führt dem Körper die erforderliche Insulinmenge zu. Über eine Bedienoberfläche, einschliesslich einer Anzeigeeinheit (Display) und Tasten, kann der Patient die Insulinpumpe einstellen und bedienen.

Diese Patientengeräte können von den Patienten relativ unauffällig und diskret am Körper getragen werden. Im Laufe eines Tages müssen die Patienten die Geräte gelegentlich einstellen oder die Einstellungen überprüfen, was unter Umständen weniger diskret möglich ist.

### Darstellung der Erfindung

Es stellt sich daher die Aufgabe, es den Patienten zu ermöglichen, die Patientengeräte diskreter einstellen und überprüfen zu können. Um dies zu ermöglichen ist in den hier beschriebenen Ausführungsbeispielen eines Patientensystems die Bedienoberfläche des Patientengerätes vom diesem abgesetzt darstellbar, zum Beispiel auf einem vom Patientengerät entfernt angeordneten Gerät. Der Patient kann dieses Gerät, das beispielsweise Bestandteil eines Mobiltelefons sein kann, komfortabel und diskret bedienen.

Ein Aspekt betrifft ein Patientensystem, das ein am Körper eines Patienten tragbares erstes Patientengerät und ein vom Patientengerät entfernt angeordnetes zweites Patientengerät, hier auch Bediengerät genannt, hat. Jedes Patientengerät hat ein Kommunikationsmodul für eine Datenübertragung über eine Übertragungsstrecke. Das erste Patientengerät hat ein Datenverarbeitungsmodul zur Kommunikation mit einer Bedienoberfläche für das erste Patientengerät. Das zweite Patientengerät ist dabei so ausgestaltet, dass es die Bedienoberfläche für das erste tragbare Patientengerät darstellt.

Ein weiterer Aspekt betrifft ein Verfahren zum Bedienen eines Patientensystems, in dem ein am Körper eines Patienten tragbares erstes Patientengerät und ein vom ersten Patientengerät entfernt angeordnetes zweites Patientengerät über eine Übertragungsstrecke miteinander kommunizieren können. Im ersten Patientengerät wird ein Datenverarbeitungsmodul zur Kommunikation mit einer Bedienoberfläche für das erste Patientengerät betrieben. Die Bedienoberfläche betreffende Daten werden zwischen dem ersten und zweiten Patientengerät kommuniziert, und die Bedienoberfläche für das erste Patientengerät wird auf dem zweiten Patientengerät dargestellt.

Das entfernt angeordnete Gerät kann ein speziell für dieses Patientensystem vorgesehenes Gerät sein, das nur zur Darstellung der Bedienoberfläche dient. Diese Funktion des Gerätes kann aber auch in einem an sich bekannten Gerät (z. B. Mobiltelefon (Smart Phone), Computer (Desktop Computer, Pocket PC, Notebook, PDA) oder Blutzuckermessgerät) integriert sein, beispielsweise in Form einer zusätzlichen Applikation. Ein derart modifiziertes Gerät hat demnach seine eigentliche Funktion, z. B. die eines Mobiltelefons, und zusätzlich die Funktion eines Bediengerätes für das Patientengerät.

Je nach Ausführung kann die Bedienoberfläche ganz oder teilweise in dem Bediengerät integriert werden. Das evtl. unerwünschte Hantieren direkt am Patientengerät ist somit nicht nötig. Das Patientengerät kann dadurch auch kleiner sein, da nur noch eine minimale Bedienoberfläche, beispielsweise ein kleineres Display, im Patientengerät erforderlich ist. Das Display kann in einem Ausführungsbeispiel auch gänzlich entfallen. Ein solches Patientengerät kann damit noch diskreter vom Patienten getragen werden.

Da die Bedienoberfläche extern dargestellt wird, unterliegt die Grösse der Darstellung der Bedienoberfläche nicht mehr den Grössenbeschränkungen des Patientengerätes. So kann die Bedienoberfläche beispielsweise auf einem grösseren Display (z. B. auf einem Mobiltelefon (Smart Phone) oder einem Computer (Desktop Computer, Pocket PC, Notebook, PDA)) dargestellt werden. Vor allem Diabetespatienten, die oft auch eine eingeschränkte Sehfähigkeit haben, können die grössere Darstellung besser erkennen.

Das hier beschriebene Bediengerät hat mindestens die gleiche Funktionalität wie die Bedienoberfläche des Patientengerätes. Das Bediengerät kann je nach Ausführungsbeispiel und Anwendung als Fernbedienung, Monitor und Statusanzeige für das Patientengerät dienen. Damit kann es auch zum Modifizieren von Parametern und Einstellungen des Patientengerätes dienen.

### Kurze Beschreibung der Zeichnungen

Weitere Ausgestaltungen, Vorteile, neue Eigenschaften und Anwendungen der Erfindung ergeben sich aus der folgenden detaillierten Beschreibung unter Einbezug der Zeichnungen. In den Zeichnungen haben gleiche Elemente die gleichen Bezugszeichen. In den Zeichnungen zeigen:
Fig. 1 eine schematische Darstellung eines Ausführungsbeispiels eines Patientensystems mit einem Patientengerät und einem Bediengerät,
Fig. 2 das Patientensystem aus Fig. 1 mit Details des Patientengerätes und des Bediengerätes, und
Fig. 3 eine schematische Darstellung einer Anzeigeeinheit.

### Wege zur Ausführung der Erfindung

Die verschiedenen Wege zur Ausführung der Erfindung sind im Folgenden ohne Einschränkung des Schutzbereiches mit Bezug auf eine Insulinpumpe beschrieben. Eine Insulinpumpe ist jedoch nur ein Beispiel für ein Patientengerät, das Patienten möglichst diskret zu tragen und zu bedienen wünschen. Pumpen für andere Medikamente, Glukosemessgeräte, Laktatmessgeräte, Blutdruckmessgeräte und EKG Geräte sind weitere Beispiele für Patientengeräte.

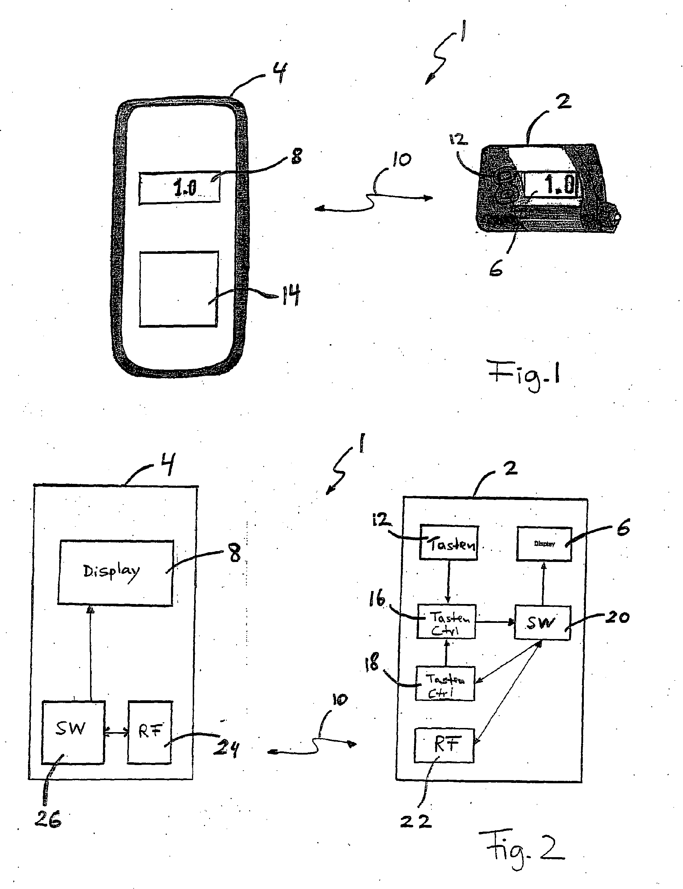

Figur 1 zeigt eine schematische Darstellung eines Ausführungsbeispiels eines Patientensystems 1 mit einem ersten Patientengerät 2 und einem zweiten Patientengerät 4 oder Bediengerät 4. Das Patientengerät 2 hat eine Anzeigeeinheit 6, im Folgenden Display 6 genannt, und Bedientasten 12. Das Bediengerät 4 hat ebenfalls eine Anzeigeeinheit 8, im Folgenden auch Display 8 genannt, und ein Tastenfeld 14.

Das Display 6 und die Bedientasten 12 sind Teil der Bedienoberfläche (oft auch als Userinterface bezeichnet) des Patientengerätes 2. Die Bedienoberfläche dient allgemein zur Interaktion zwischen einem Patienten und dem Patientengerät 2 und ermöglicht u. a. die Bedienung des Patientengerätes 2 durch den Patienten. Die Bedienoberfläche stellt Information beispielsweise optisch oder akustisch dar. Je nach Zustand des Patientengerätes 2 kann der Patient auf die dargestellte Information reagieren, beispielsweise durch Drücken der Bedientasten 12, um die dargestellte Information zu bestätigen. Zur dargestellten Information gehören auch Alarme und Alarm- und Fehlermeldungen. Derartige Alarme und Meldungen können beispielsweise durch Textnachrichten, akustische Signale (Summer, auch Buzzer genannt) oder Vibrationen dem Patienten mitgeteilt werden. Die Bedienoberfläche umfasst daher auch Einrichtungen, die akustische Signale und Vibrationen erzeugen.

Das Patientengerät 2 und das Bediengerät 4 stehen über eine Übertragungstrecke 10 in Verbindung. Je nach Ausgestaltung der im Patientensystem 1 vorhandenen Geräte (2, 4) kann die Übertragungsstrecke 10 für eine drahtlose Datenübertragung ausgelegt sein. Die Bedienoberfläche des Patientengerätes 2 kann damit auf dem Bediengerät 4 dargestellt, so dass ein Patient das Patientengerät 4 auf Distanz diskret bedienen kann. Im in Figur 1 gezeigten Ausführungsbeispiel ist die Übertragungsstrecke 10 für eine drahtlose und bidirektionale Datenübertragung ausgelegt.

In einem Ausführungsbeispiel können über die Übertragungsstrecke 10 Daten gemäss dem Bluetooth® Standart (Serial Port Profile (SSP)) mit einem darüber liegenden Protokoll der Applikationsebene (Application Layer Protocol) ausgetauscht werden. Der Bluetooth Standard ist für die drahtlose Übermittlung von Sprache und Daten im Nahbereich entwickelt worden und arbeitet mit einer Übertragungsrate von bis zu 1 MBit pro Sekunde im 2.45-GHz-Bereich. In einem anderen Ausführungsbeispiel kann die Übertragung gemäss dem IrDA, WLAN (802.11x), Zigbee, Wi-Max, Mics Protokoll erfolgen.

Im gezeigten Ausführungsbeispiel ist das Patientengerät 2, wie erwähnt, eine Insulinpumpe. Das Display 6 zeigt in Figur 1 die eingestellte Basalrate (1,0) an. In anderen Ausführungsbeispielen kann das Display 6 im Normalbetrieb zusätzliche Information anzeigen, beispielsweise die Einheit der Basalrate (U/h) und die Uhrzeit. Mit den Tasten 12 kann der Patient das Patientengerät 2 menügesteuert bedienen, beispielsweise die Basalrate einstellen oder einen Bolus auslösen. Während der Bedienung des Patientengerätes 2 zeigt das Display 6 ein Benutzermenü.

Das Bediengerät 4 ist in Figur 1 in Form eines schematisch gezeigten Mobiltelefons ausgeführt. Das. Display 8 zeigt ebenfalls die am Patientengerät 2 eingestellte Basalrate (1,0) an. Wie im Folgenden noch genauer ausgeführt ist, stellt das Display 8 ein Abbild der Anzeige auf dem Display 6 dar. Über das Tastenfeld 14 und dem auf dem Display 6 dargestellten Menü kann der Patient das Patientengerät 2 aus der Entfernung bedienen. Je nach Ausgestaltung des Bediengerätes 4 (Spezialgerät oder kombiniertes Gerät) kann das Tastenfeld 14 Auswahltasten (z. B. MENÜ, SELECT, OK, AUF und AB) und eine Art Joystick aufweisen. Darüber hinaus oder alternativ kann das Bediengerät 4 verschiedene Ein- und Ausgabemethoden unterstützen, beispielsweise Funktionstasten, Touchscreen, Sprache, akustische und visuelle Signale.

Figur 2 zeigt das Patientensystem 1 aus Figur 1, wobei zusätzlich Details des Patientengerätes 2 und des Bediengerätes 4 schematisch und beispielhaft gezeigt sind. Das Patientengerät 2 hat neben dem bereits erwähnten Display 6 und den Tasten 12 verschiedene Module. Ein Datenverarbeitungsmodul 20 dient zur Kommunikation mit der Bedienoberfläche und enthält das Hauptprogramm, das die Pumpe und das Display 6 abhängig von der Tasteneingabe steuert und eine Displayanzeige aufbaut. Das Datenverarbeitungsmodul 20 erzeugt Daten zur Ansteuerung von Bildpunkten eines Displays, um darauf Information darzustellen. Dazu steht das Datenverarbeitungsmodul 20 mit mehreren Modulen 16, 18, 22 direkt oder indirekt in Verbindung. Das Modul 16 verarbeitet die Tasteneingaben, die am Patientengerät 2 mit Hilfe der Tasten 12 oder am Bediengerät 4 erfolgen. Die am Bediengerät 4 erfolgten Tasteneingaben erhält das Modul 16 vorverarbeitet vom Modul 18. Das Modul 18 erhält die am Bediengerät 4 erfolgte Tasteneingabe als dieser Eingabe entsprechende Datensignale vom Datenverarbeitungsmodul 20, das mit dem Modul 22, oder Kommunikationsmodul 22, in Verbindung steht.

In einem Ausführungsbeispiel sendet und empfängt das Kommunikationsmodul 22 Daten gemäss dem Bluetooth® Standart. Das Kommunikationsmodul 22 sendet beispielsweise die aktuelle Displayanzeige Bildpunkt für Bildpunkt (Pixel für Pixel) zum Bediengerät 4. Das Kommunikationsmodul 22 empfängt ausserdem Daten, die beispielsweise einer durch Tastendruck ausgewählten Funktion entsprechen, vom Bediengerät 4. Diese Daten werden anschiessend durch das Modul 18 verarbeitet.

Das Bediengerät 4 hat neben dem Display auch ein Programmmodul 26 und ein Kommunikationsmodul 24, das nach dem gleichen Standard/Protokoll arbeitet wie das Kommunikationsmodul 22 und zur Kommunikation mit dem Kommunikationsmodul 22 des Patientengerätes 2 dient. Das Programmmodul 26 steuert allgemein das Bediengerät 4 und damit auch das Display 8.

Das Patientensystem 1 kann als Client-Server System aufgefasst werden, wobei das Patientengerät 2 der Server ist und das Bediengerät 4 der Client ist. Das Patientensystem 1 kann in einem "Push" Modus oder einem "Request" Modus betrieben werden. Im Push Modus verhält sich das Patientengerät 2 wie ein "Push" Server. Während der Benutzung kann sich die Information auf dem Patientengerät 2 ändern, beispielsweise wegen einer Fehlermeldung oder eines Alarms (z. B. ein Okklusionsalarm). Wenn sich die Information auf dem Patientengerät 2 ändert (z. B. Displayanzeige, Alarm (Summer, Vibration)), werden die entsprechenden Daten automatisch vom Patientengerät 2 zum Bediengerät 4 gesendet und dort umgesetzt, beispielsweise Information dargestellt oder ein Alarm aktiviert, d. h. das Bediengerät 4 fängt zum Beispiel an zu summen oder zu vibrieren. Das Bediengerät 4 ist somit passiv und wartet, bis ihm die aktuellsten Informationen gesendet werden.

Im "Request" Modus ist das Patientengerät 2 passiv, d. h. das Patientengerät 2 sendet nicht von sich aus Daten zum Bediengerät 4. Das Bediengerät 4 muss in diesem Modus die Informationen selbständig vom Patientengerät 2 abfragen, beispielsweise periodisch.

Das im Patientensystem 1 implementierte Protokoll der Applikationsebenen ist Kommando orientiert. In einem Ausführungsbeispiel können beispielsweise folgende Kommandos vom Bediengerät 4 zum Patientengerät 2 gesendet werden: Fernbedienmodus starten/beenden, Displaydaten abfragen, Infotext abfragen, Summerdaten abfragen und Vibrationsdaten abfragen, Tastendrücke senden und Modus setzen (Push oder Request). In ähnlicher Weise können beispielsweise folgende Kommandos vom Patientengerät 2 zum Bediengerät 4 gesendet werden: Displaydaten, Infotext, Summerdaten und Vibrationsdaten.

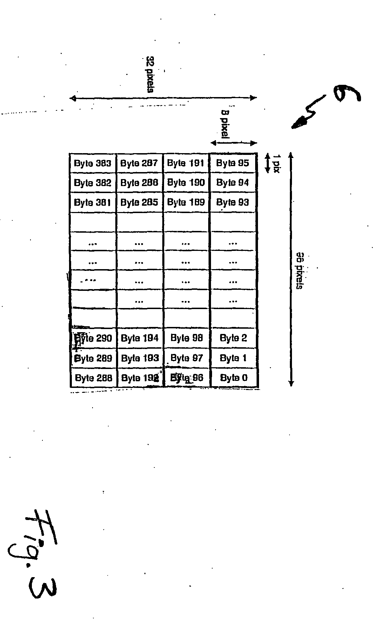

Wenn das Patientengerät 2 vom Bediengerät 4 das Kommando "Displaydaten abfragen" empfängt, oder wenn sich im "Push" Mode der Displayinhalt geändert hat, sendet das Patientengerät 2 mit dem Kommando "Displaydaten" den aktuellen Inhalt des Displays 6 zum Bediengerät 4. Figur 3 zeigt dazu eine schematische Darstellung des Displays 6, das aus einzelnen Bildpunkten, sogenannten Pixel aufgebaut ist. Im gezeigten Ausführungsbeispiel hat das Display 32x96 Pixels, wobei acht Pixel jeweils in einem Byte codiert sein können. Bei einem monochromen Display ist jedes Pixel entweder schwarz oder weiss. Damit ergeben sich 384 Bytes ((32x96)/8) pro Displayinhalt. Wie im Ausführungsbeispiel der Figur 3 gezeigt ist, besteht das Display 6 aus vier Reihen, wobei jede Reihe eine Fläche von 96x8 Pixel abdeckt.

Das Patientengerät 2 sendet den Displayinhalt Reihe für Reihe zum Bediengerät 4. In einem Ausführungsbeispiel sendet das Patientengerät 2 nach dem Empfang eines ersten Kommandos "Displaydaten anzeigen" (RequestDisplayData (0)) die erste Reihe (Bytes 0-95) und dann nach jedem weiteren Kommando die jeweils nächste Reihe. Sind alle vier Reihen gesendet, antwortet das Patientengerät 2 auf das nächste Kommando (RequestDisplay-Data (4)) mit dem Senden der gesamten Displaydaten (384 Bytes).

Das Bediengerät 4 empfängt daher vom Patientengerät 2 ein Abbild der aktuellen Displayanzeige und stellt dieses Abbild Pixel für Pixel auf seinem Display 8 dar. In der in Figur 1 gezeigten Minimalausführung ist die Anzeige auf die Anzeige des Wertes 1,0 beschränkt. In einem Ausführungsbeispiel kann das Abbild auf dem Display 8 durch zusätzliche Information erweitert werden, beispielsweise durch die Anzeige der Einheit der Basalrate in U/h und der aktuellen Zeit. Die Anzeige auf dem Bediengerät 4 ist daher umfangreicher als auf dem Patientengerät 2. In einer "Minimalausführung" des Patientengerätes 2 kann das Display 6 daher relativ klein sein oder gänzlich wegfallen.

In einem weiteren Ausführungsbeispiel eines Patientensystems kann das Bediengerät 4 mit einem Glukosemessgerät als Patientengerät kombiniert sein. Ähnlich wie beim oben beschriebenen Patientengerät 2 kann die Bedienoberfläche des Glukosemessgerätes auf dem Bediengerät 4 dargestellt werden. Ist das Bediengerät 4 beispielsweise kombiniert mit einem Mobiltelefon kann das Glukosemessgerät drahtlos oder über eine Schnittstelleneinrichtung daran gekoppelt werden. Das Glukosemessgerät kann dabei ohne ein eigenes Display auskommen. Ein gemessener Glukosewert kann dann auf dem Display 8 angezeigt werden. Ist in einem Ausführungsbeispiel das oben beschriebenen Patientengerät 2 (Insulinpumpe) teil diese Patientensystems kann der Patient diesen Wert bequem ablesen und wenn nötig am Bediengerät 4 die Pumpeneinstellung diskret ändern.

Ist das Bediengerät 4 in einem Mobiltelefon integriert kann in einem Ausführungsbeispiel das Mobiltelefon beispielsweise die Insulinpumpenparameter und den gemessenen Glukosewert auswerten. Ergibt die Auswertung, dass ein medizinisch bedenklicher Zustand vorliegt, kann das Mobiltelefon über das Mobilfunknetz beispielsweise einen Alarm an eine Notfallzentrale oder einen Arzt senden. Diese können dann entsprechende Massnahmen einleiten, um den Patienten medizinisch zu versorgen.

Die im vorhergehenden beschriebenen Ausführungsbeispiele eines Patientensystems mit mindestens einem Patientengerät und einem Bediengerät ermöglichen die vollständige oder nur teilweise Auslagerung der Bedienoberfläche des Patientengerätes in das Bediengerät. Dadurch kann der Patient aus der Entfernung das Patientengerät bequem und diskret einstellen und bedienen. Die Freiheit, das Patientengerät 4 direkt zu bedienen bleibt dabei jedoch erhalten.

## Patentansprüche

1. Patientensystem (1) mit einem am Körper eines Patienten tragbaren ersten Patientengerät (2) und einem vom ersten Patientengerät (2) entfernt angeordneten zweiten Patientengerät (4), wobei jedes Patientengerät (2, 4) ein Kommunikationsmodul (22, 24) für eine Datenübertragung über eine Übertragungsstrecke (10) hat, wobei das erste Patientengerät (2) ein Datenverarbeitungsmodul (20) zur Kommunikation mit einer Bedienoberfläche für das erste Patientengerät (2) hat, und wobei das zweite Patientengerät (4) so ausgestaltet ist, dass es die Bedienoberfläche für das erste tragbare Patientengerät (2) darstellt.

2. Patientensystem (1) nach Anspruch 1, bei dem das Datenverarbeitungsmodul (20) Daten zur Ansteuerung von Bildpunkten erzeugt, um auf einer Anzeigeeinheit (6, 8) Information darzustellen, und bei dem das Kommunikationsmodul (22) des ersten Patientengerätes (2) diese Daten zum Kommunikationsmodul (24) des zweiten Patientengerätes (4) sendet, um auf einer Anzeigeeinheit (8) des zweiten Patientengerätes (4) die Information darzustellen.

3. Patientensystem (1) nach Anspruch 2, bei dem das erste Patientengerät (2) ebenfalls eine Anzeigeeinheit (6) hat und die Informationsanzeige darstellt.

4. Patientensystem (1) nach Anspruch 3, bei dem das zweite Patientengerät (4) zusätzlich zur Informationsanzeige weitere Information, insbesondere Alarme und Alarm- und Fehlermeldungen, darstellt.

5. Patientensystem (1) nach einem der vorhergehenden Ansprüche, bei dem das Datenverarbeitungsmodul (20) Daten erzeugt, um im zweiten Patientengerät (4) eine Einrichtung zur Erzeugung eines akustischen Alarms oder eines Vibrationsalarms anzusteuern.

6. Patientensystem (1) nach einem der vorhergehenden Ansprüche, bei dem die auf dem zweiten Patientengerät (4) dargestellte Bedienoberfläche mindestens eine Eingabevorrichtung (14) enthält, und bei dem das Kommunikationsmodul (24) des zweiten Patientengerätes (4) einer Eingabe entsprechende Daten zum ersten Patientengerät (2) sendet.

7. Patientensystem (1) nach einem der vorhergehenden Ansprüche, beim dem die Kommunikationsmodule (22, 24) so ausgestaltet sind, dass sie Funksignale senden und empfangen können.

8. Patientensystem (1) nach einem der vorhergehenden Ansprüche, bei dem das zweite Patientengerät (4) ein Mobiltelefon oder ein Computer ist.

9. Verfahren zum Bedienen eines Patientensystems (1), in dem ein am Körper eines Patienten tragbares erstes Patientengerät (2) und ein vom ersten Patientengerät (2) entfernt angeordnetes zweites Patientengerät (4) über eine Übertragungsstrecke (10) miteinander kommunizieren können,
bei dem im ersten Patientengerät (2) ein Datenverarbeitungsmodul (20) zur Kommunikation mit einer Bedienoberfläche für das erste Patientengerät (2) betrieben wird,
bei dem die Bedienoberfläche betreffende Daten zwischen dem ersten und zweiten Patientengerät (2, 4) kommuniziert werden, und
bei dem die Bedienoberfläche für das erste Patientengerät (2) auf dem zweiten Patientengerät (4) dargestellt wird.

10. Verfahren nach Anspruch 9, bei dem vom Datenverarbeitungsmodul (20) Daten zur Ansteuerung von Bildpunkten erzeugt werden, um auf einer Anzeigeeinheit (6, 8) Information darzustellen, und bei dem diese Daten zum zweiten Patientengerät (4) gesendet werden, um auf einer Anzeigeeinheit (8) des zweiten Patientengerätes (4) die Information darzustellen.

11. Verfahren nach Anspruch 10, bei dem mittels der auf dem zweiten Patientengerät (4) dargestellten Bedienoberfläche mindestens eine Eingabe vorgenommen wird, und bei dem dieser Eingabe entsprechende Daten zum ersten Patientengerät (2) gesendet werden.

12. Verfahren nach einem der Ansprüche 9 bis 11, bei dem im ersten Patientengerät (2) Daten erzeugt werden, um im zweiten Patientengerät (4) eine Einrichtung zur Erzeugung eines akustischen Alarms oder eines Vibrationsalarms anzusteuern.

13. Verfahren nach einem der Ansprüche 9 bis 12, bei dem die die Bedienoberfläche betreffenden Daten vom ersten Patientengerät (2) zum zweiten Patientengerät (4) gesendet werden, sobald sich eine das erste Patientengerät (2) betreffende Information ändert.

14. Verfahren nach einem der Ansprüche 9 bis 12, bei dem mindestens ein Kommando vom zweiten Patientengerät (4) ausgesendet wird, um die Bedienoberfläche betreffende Daten vom ersten Patientengerät (4) anzufordern.

15. Verfahren nach einem der Ansprüche 9 bis 14, bei dem die Bedienoberfläche betreffende Daten vom ersten Patientengerät (2) byteweise ausgesendet werden.

16. Verfahren nach Anspruch 15, bei dem jedes Byte einer Fläche auf einem Display (6) entspricht, um so eine Displayanzeige des ersten Patientengerätes (2) auf einem Display (8) des zweiten Patientengerätes (4) abzubilden.
